# EUROPEAN PATENT SPECIFICATION

(11) **EP 2 433 927 B1**
(45) Date of publication and mention of the grant of the patent: **20.11.2013**
(21) Application number: 09844829.3
(22) Date of filing: 30.12.2009
(51) Int. Cl.: C07D 209/86, C07C 319/20, C07C 323/47, C07C 251/64, C08F 2/46, G03F 7/031, C08F 2/50

(54) **KETOXIME ESTER PHOTOINITIATOR**
KETOXINESTER ALS PHOTOINITIATOR
PHOTOINITIATEUR À BASE D'ESTER CÉTOXIME

(30) Priority: 19.05.2009 CN 200910027707
(43) Date of publication of application: 28.03.2012
(73) Proprietor: Changzhou Tronly New Electronic Materials Co., Ltd, Jiangsu 213011 (CN)
(72) Inventor: QIAN, Xiaochun, Jiangsu 213011 (CN)
(74) Representative: Hart-Davis, Jason
(86) International application number: PCT/CN2009/076303
(87) International publication number: WO 2010/133077

(56) References cited:
- EP-A2- 2 055 746
- WO-A1-2008/078686
- WO-A2-2004/050653
- CN-A- 1 241 562
- CN-A- 101 565 472
- US-B1- 6 596 445

## Description

### FIELD OF THE INTENTION

The present invention relates to the technical field of ketoxime ester compounds and, more particularly, to a ketoxime ester photoinitiator used for photo curing materials.

### BACKGROUND OF THE INTENTION

In order to make photocurable materials (photocurable coatings, ink, photoresist, CF, BM), which are comprised mainly of unsaturated resin and the monomeric material thereof, take place polymerization and solidification by irradiating them with ultraviolet rays, X-rays or laser light, photoinitiators or sensitizers must be added. By irradiating photocurable materials with ultraviolet rays, X-rays or laser light of a certain wavelength, the added photoinitiators or sensitizers can produce active groups, and initiate unsaturated groups in the photocurable materials to take place polymerization and solidification.

In photocurable materials, some traditional photoinitiators are widely used, for example, benzoin derivatives, benzil ketals, α, α'-dialkoxy acetophenones, α-hydroxy alkyl phenones, α-amino alkyl phenones, acylphosphine oxides, benzophenone/diphenylamine series, Michler's ketone, thioxanthone/thioxanthone carboxylic amide, amine hardener, aryl diazonium salt, aryl iodonium salt, aryl sulfonium salt, ferrocene, titanocene, hexaphenylbiimidazols, triazines and traditional oxime esters and so on. Because these traditional photoinitiators have demerits more or less sensitivity is low (both polymerization velocity conversion are low); solubility is bad (transparency is low and there is too much photoresist residue); oxygen gas has a great effect on photo curing; storage stability is bad and so on. Therefore the use of traditional photoinitiators and photosensitive materials is considerably restricted, the performance of photosensitive materials is also greatly affected, especially, these traditional photoinitiators not fulfill the requirements for the manufacture of BM and CF which are the key parts for new type of large-screen LCD, The emergence of a new oxime ester photoinitiator has solved above-mentioned problems to a great extent.

The photochemical performance of oxide ester compounds first emerged from the literature reported by A. Wemer and A. Piguet in Ber. Dtsch. 1904, 37, 4295; while the application of oxime ester compounds as photoinitiator first emerged from the literature reported by G. A. Delzenne, U. Laridon and H. Peeters in European Polymer Journal, 1970, 6, 933-943, the trade names are DE-OS 179508 and Agfa-Gevaert AG. The oxime ester photoinitiator which once be produced commercially and used more widely is Quantacure PDO; structural formula for Quantacure PDO is shown below.

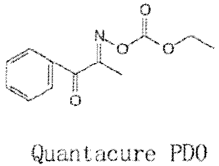

Although these traditional oxime ester photoinitiators have high initiator activity, their thermal stability is low, and therefore they have been eliminated gradually. Resurrection of oxime ester photoinitiators first emerged from the literature reported by R. Mallivia et al in J. Photochem. Photobiol. A: Chemistry 2001, 138, 193 and the literature reported by L. Lavalée et al in J. Photochem. Photobiol. A: Chemistry 2002, 151, 27. Because of the introduction of diphenyl-sulfide and carbazolyl, which have bigger conjugated system and stronger intramolecular electronic transfer characteristics, stability and photosensitivity of the oxime ester compound are greatly improved. Two representative oxime ester photoinitiators which are widely used at present are OXE-1 and OXE-2; structural formulae for OXE-1 and OXE-2 are separately shown below.

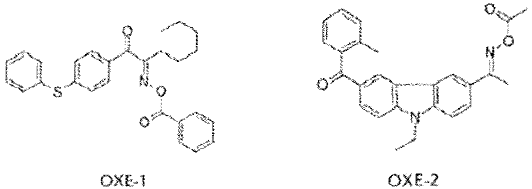

Wherein, OXE-1 is a typical ketoxime ester photoinitiator which is mainly used in the manufacture of BM RGB of large-screen LCD. Its price is very high, and the structural formulae have been protected by foreign companies' patents, publication numbers of which are CN 99108598 and CN 02811675. The disclosed synthesis methods of the compound having said structural formulae are very complicated, and demand high cost. The products having said structure have the defects of discontented using capability and thermal stability.

### DETAILED DESCRIPTION OF THE INVENTION

The present invention solves the problems that the existing ketoxime ester photoinitiator such as OXE-1 has bad application performance and bad thermal stability. It is an object of the invention to provide a new ketoxime ester photoinitiator which has better thermal stability and a preparing method thereof.

The technical solution, which the inventor adopts to solve the above-mentioned technical problem, is a compound suitable as ketoxime ester photoinitiator shown in the following general formula:

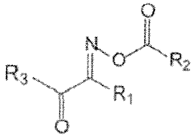

Wherein, the group R₁ is 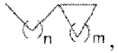 wherein, n is the integer of 1∼4, m is the integer of 1∼6; R₂ is C1-8 alkyl, phenyl, substituted phenyl, benzyl, or substituted benzyl; R₃ is diphenylthioether group, substituted diphenylthioether group, carbazyl or substituted carbazyl.

The process for preparing the ketoxime ester photoinitiator of the present invention comprises the following steps:
a. Preparation of intermediate I: diphenylthioether, substituted diphenylthioether, carbazole or substituted carbazole as a starting material is mixed with an acyl halide compound having R₁ group, with the participant of aluminum chloride or zinc chloride, an Friedel-Crafts Reaction is carried out, by which a ketoxime ester photoinitiator intermediate I is obtained.
b. Preparation of intermediate II: the intermediate I is oxidized by isoamyl nitrite, with the catalysis of hydrogen chloride, sodium alcoholate or potassium alcoholate, a hydroxylamine compound, i.e. intermediate II, is obtained.
c. Preparation of ketoxime ester photoinitiator: the intermediate II is esterified by acyl halide compound having R₂ group or anhydride having R₂ group to prepare ketoxime ester photoinitiator; the reaction process is shown below:

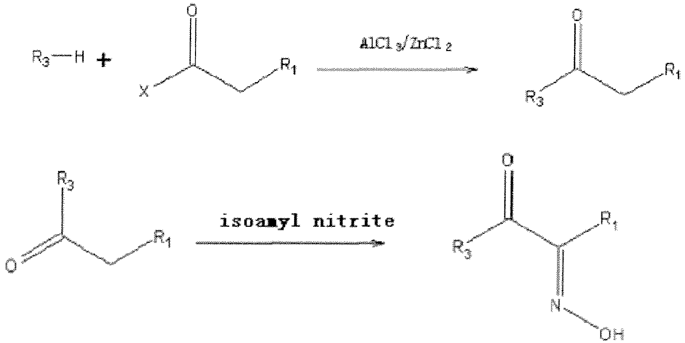

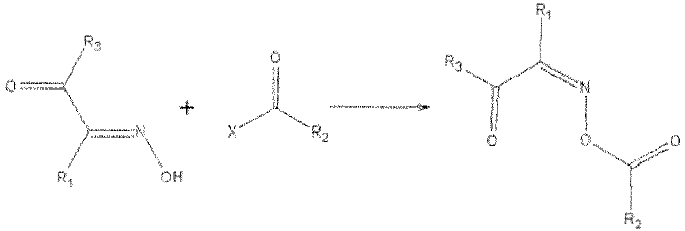

In the present invention, the structure of the acyl halide compound having R₁ group is 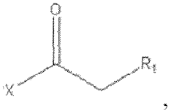 the structure of the intermediate I is 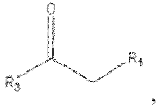 the structure of the intermediate II is 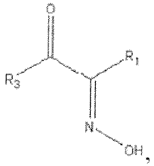 the structure of the acyl halide compound having R₂ group is 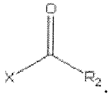 Wherein, the structure of R₁ is 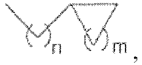 wherein, n is the integer of 1∼4, m is the integer of 1∼6; R₃ is diphenylthioether group, substituted diphenylthioether group, carbazyl or substituted carbazyl.

With respect to the step a, the specific process of preparing intermediate I is as follows: the starting material,AlCl₃, and organic solvent A are mixed by agitating, pump the argon gas into the mixture to protect the reaction system, cool down them with an ice bath; when the temperature falls to 0°C, begin to add dropwise a mixture solution of acyl halide compound having R₁ group and organic solvent A for 1.5∼2 hours, while maintain a constant temperature of 10°C or less; then the temperature rises to 5∼35 °C, continue to agitate the reaction mixture for 1.5∼3 hours; finally, discharge the materials, after post-treatment the intermediate I as a sheet white solid is obtained. Wherein, the optimal mole ratio of the material, AlCl₃ and the acyl halide compound having R₁ group is 1:1:1.

Said organic solvent A of the present invention is dichloroethane, dichloromethane or carbon tetrachloride.

With respect to the step b, the process of preparing intermediate II is as follows: add hydrogen chloride, sodium alcoholate or potassium alcoholate into a mixture solution of the intermediate I obtained from the step a, the organic solvent B and isoamyl nitrite, continue to agitate the reaction mixture for 5 hours at room temperature. After post-treatment the intermediate II as a powdery white solid is obtained. Wherein, the optimal mole ratio of the intermediate I to isoamyl nitrite is 1:1.6.

Said organic solvent B of the present invention is dichloroethane or tetrahydrofuran, optimally is tetrahydrofuran.

With respect to the step c, the process of preparing ketoxime ester photoinitiator is as follows: the intermediate II, dichloroethane and triethylamine are mixed by agitating, cool down them with an ice bath; when the temperature falls to 0°C, begin to add dropwise a mixture solution of acyl halide compound having R₂ group and dichloroethane for 1∼1.5 hours, continue to agitate the reaction mixture for 1∼2 hours; then add cold water dropwise to the materials; separate out organic liquid layer of the materials with a separating funnel; wash it with 5% sodium bicarbonate one time, then wash it with water till the organic liquid layer have pH 7, furthermore, it with diluted hydrochloric acid solution one time, and then wash it with water three times; subsequently, in order to remove water, pour anhydrous magnesium sulfate into it; distill out the solvents via rotary evaporation method to obtain a liquid; pour methanol into it to obtain a white solid product as a precipitate.

With respect to the formula of the present invention, R₁ is cyclopentylmethylene, R₃ is 6-o-methyl-benzoyl-9-ethylcarbazyl group, R₂ is phenyl, the structural formula is 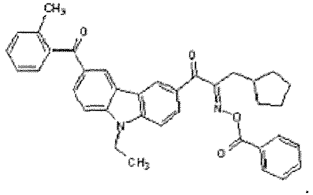 The compound which has a biggish absorption peak at 365nm of UV absorption spectrum can be used as photoinitiator in LED cold light source.

The structural formula of the present invention is also applicable to symmetric or dissymmetric multi-ketoxime ester structure, i.e. R₃ group may have substituents and ketoxime ester structure.

Beneficial effects: with respect to the invention, the ketoxime ester compounds have same or similar UV absorption spectrum with that of OXE-1, in the case of having same weight concentration. The thermal stability of the ketoxime ester compounds is obviously better than that of OXE-1. Partial structure thereof show obvious red shift in UV absorption spectrum compared with OXE-1, it has biggish absorption peak at 365nm. So it can be realized that LED cold light source be used as initiate light source. The practical performance (sensitivity, thermal stability, solubility) of the new ketoxime ester of the present invention is superior to that of OXE-1.

### BRIEF DESCRIPTION OF THE DRAWINGS

Hereinafter, the invention will be concretely described with embodiments and drawings.

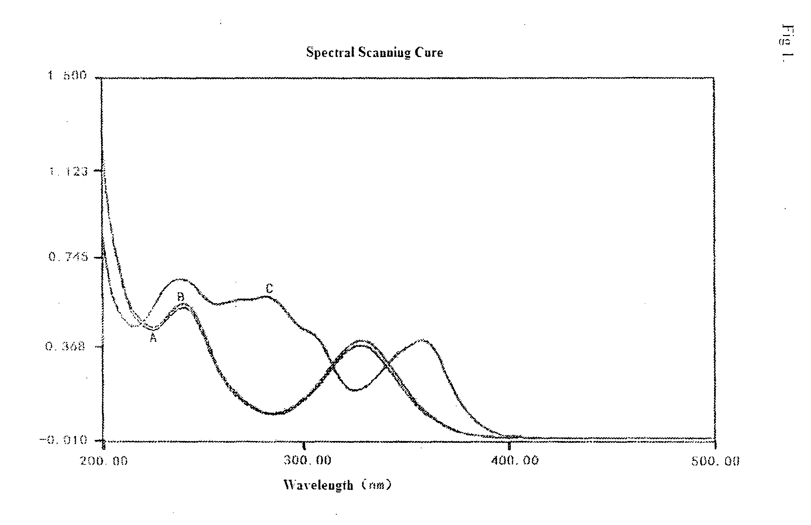

Fig. 1 is the comparison of UV absorption spectrum of the 1-(6-o-methylbenzoyl-9-ethylcarbazole-3-yl)-(3-cyclopentyl)-propane-1,2-dione-2-benzoic acid oxime ester and 1-(4-phenylthiophenyl)-(3-cyclopentyl)-propane-1,2-dione-2-benzoic acid oxime ester of the present invention, and 1-(4-plienylthiophenyl)-n-octane-1,2-dione-2-benzoic acid oxime ester of the prior art. Wherein, A is the UV absorption spectrum of 1-(4-phenylthiophenyl)-n-octane-1.2-dione-2-benzoic acid oxime ester, B is the UV absorption spectrum of 1-(4-phenylthiophenyl)-(3-cyclopentyl)-propane-1,2-dione-1-benzoic acid oxime ester, and C is the UV absorption spectrum of 1-(6-o-methylbenzoyl-9-ethylcarbazole-3-yl)-(3-cyclopentyl)-propane-1,2-dione-2-benzoic acid oxime ester.
Fig. 2 is the ¹HNMR spectrogram of 1-(4-phenylthiophenyl)-(3-cyclopentyl)-propane-1,2-dione-2-benzoic acid oxime ester.
Fig. 3 is the ¹HNMR spectrogram of 1-(6-o-methylbenzoyl-9-ethylcarbazole-3-yl)-(3-cyclopentyl)-propane-1,2-dione-2-benzoic acid oxime ester.

### DETAILED DESCRIPTION

### EXAMPLES

### Example 1:

### The process for preparing 1-(4-phenylthiophenyl)-(3-cyclopentyl)-propane-1,2-dione-2-benzoic acid oxime ester

### Step 1: Preparation of 1-(4-phenylthiophenyl)-(3-cyclopentyl)-prop-1-one

Charge 0.2 mol diphenylthioether, 0.22mol AlCl₃ (fine grinding), 150 ml dichloroethane into a 500 ml four-neck flask, carry on agitating, in order to protect the materials in the flask, pump the argon gas into the flask, cool down them with an ice bath; when the temperature falls to 0°C, begin to add dropwise the solution of 0.22 mol cyclopentylpropionyl chloride and 42 g dichloroethane dissolved in cyclopentyl to the materials, while maintain a constant temperature of 10°C or less, add dropwise the above-mentioned solution to the materials for about 1.5 hours, then the temperature rises to 15°C, continue to agitate the materials in the flask for 2 hours; Finally, discharge the materials.

### Post-treatment:

Under agitation, gradually add the above-mentioned materials into the diluted solution of hydrochloric acid which is prepared by mixing 65 ml of concentrated hydrochloric acid with 400 g of ice; separate out the lower layer of the mixture with a separating funnel, and extract the upper layer of the mixture with 50 ml of dichloroethane; combine the lower layer materials with the extracted materials; wash it with the solution of sodium bicarbonate, which is prepared by mixing 10 g of sodium bicarbonate, with 200 g of water, furthermore, wash it with 200 ml of water three times, till the mixture have pH 7; subsequently, remove water with 60 g of anhydrous magnesium sulfate, then remove dichloromethane via rotary evaporation method; after the evaporation, the crude product in distilling flask is a powdery solid, pour the powdery solid into 200 ml of light petroleum which is distilled at normal pressure, obtain a mixture; filtrate the mixture with air pump filtration to obtain a filter cake; pour the filter cake into 150 ml of anhydrous ethanol to obtain a mixture; heat the mixture to reflux, then, cool it down to room temperature, furthermore, freeze it for 2 hours; finally, filtrate it with air pump filtration to obtain 1-(4-phenylthiophenyl)-(3-cyclopentyl)-prop-1-one as sheet write solid; dry the sheet write solid in an oven at 50°C for 2 hours to obtain the product. The weight is 53.4 g; the yield is equal to 86 %; the purity is more than 98 %.

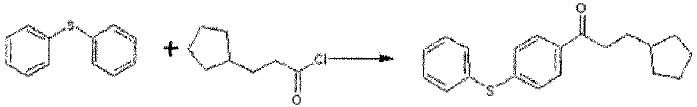

### Step 2: Preparation of 1-(4-phenylthio-phenyl)-(3-cyclopentyl)-propane-1,2-dione-2-oxime

Charge 40g of the product obtained in the step 1, 400 g of tetrahydrofuran, 200 g of concentrated hydrochloric acid, and 24.2 g of isoamyl nitrite into a 500 ml four-neck flask, agitate at room temperature for 5 hours; and then discharge the materials; during the reaction process, large quantities of the materials were precipitated.

### Post-treatment:

Pour the above-mentioned materials into a big beaker, furthermore, pour 1000 g of water into the same beaker, carry on agitating to obtain a mixture; leave the mixture at rest overnight; then the mixture is separated to two layers, separate the layers to obtain viscous yellow liquid, extract it with dichloroethane; in order to remove water from the mixture, pour 50 g of anhydrous magnesium sulfate into the materials; carry on filtrating to obtain a filtrate; evaporate the filtrate via rotary evaporation method; after the evaporation, oily viscous material is obtained in distilling flask, pour the oily viscous material into 150 ml of petroleum ether, carry on agitating to obtain a precipitate; filtrate the precipitate with air pump filtration to obtain a powdery white solid, dry the powdery white solid in an oven at 60°C for 5 hours to obtain the product. The weight is 29.4 g; the yield is equal to 67.2 %; and the purity is more than 98 %.

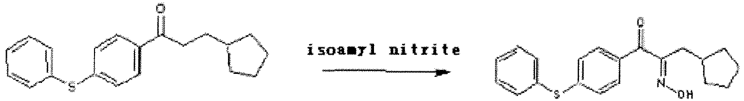

### Step 3: Preparation of 1-(4-phenylthiophenyl)-(3-cyclopentyl)-propane-1,2-dione-2-benzoic acid oxime ester

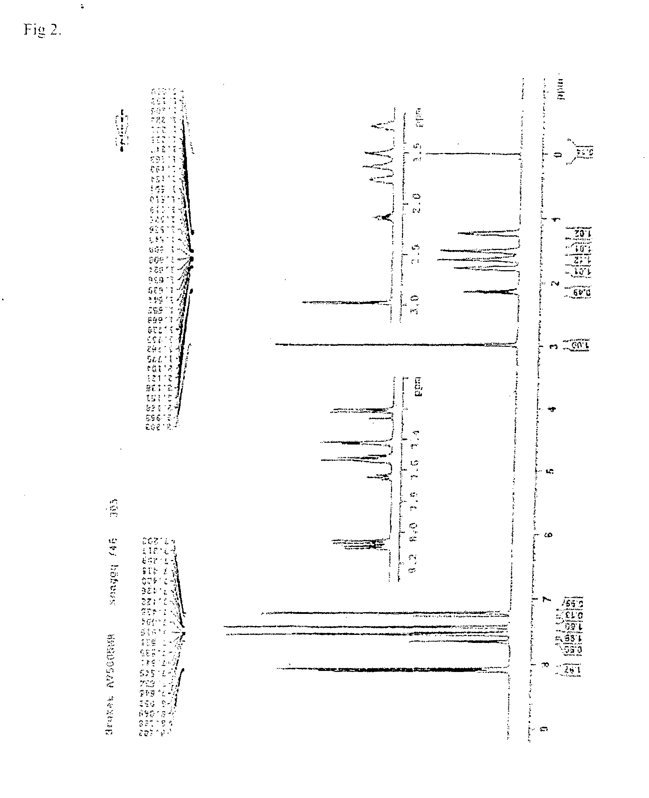

Charge 33 g of the product obtained in the step 2, 350 ml of dichloroethane and 12.7 g of triethylamine into a 1000 ml four-neck flask, carry on agitating, cool down the materials in the flask with an ice bath; when the temperature falls to 0°C, begin to add dropwise the solution of 15.7 g of benzoyl chloride and 15 g of dichloroethane to the materials for about 1.5 hours; continue to agitate the materials for 1 hour; then add dropwise 500 ml of cold water to the materials; separate out organic liquid layer of the materials with a separating funnel, wash it with 200 ml of 5% sodium bicarbonate one time, furthermore, wash it with 200 ml of water two times till the organic liquid layer have pH 7; then wash it with the diluted solution of hydrochloric acid one time, which is prepared by mixing 20 g of concentrated hydrochloric acid with 400 ml of water, furthermore, wash it with 200 ml of water three times; subsequently, in order to remove water, pour 100 g of anhydrous magnesium sulfate into it; distill out the solvents via rotary evaporation method to obtain a viscous liquid; add proper quantity of methanol into the viscous liquid, then a white solid product as a precipitate is obtained; finally, carry on filtrating and drying to obtain a final product. The weight is 33 g; the yield is equal to 76.5 %; and the purity is more than 98 %. According to the ¹HNMR spectrogram as shown in Fig. 2, it can be deduced that the structural formula of the final product of this example is as follows:

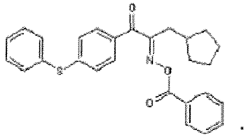

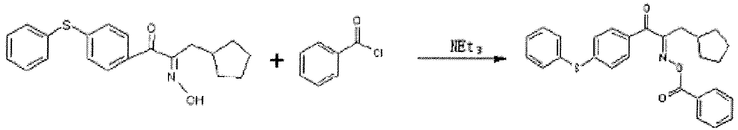

### Example 2:

### The process for preparing 1-(6-o-methylbenzoyl-9-ethylcarbazole-3-yl)-(3-cyclopentyl)-propane-1,2-dione-2-benzoic acid oxime ester

### Step 1: Preparation of 1-(6-o-methyl-benzoyl-9-ethylcarbazole-3-yl)-(3-cyclopentyl)-prop-1-one

Charge 39.0 g of N-ethyl carbazole, 25.3g of AlCl₃ (fine grinding) and 150 ml of dichloroethane into a 500 ml four-neck flask, carry on agitating, in order to protect the materials in the flask, pump the argon gas into the flask, cool down them with an ice bath; when the temperature falls to 0°C, begin to add dropwise the solution of 25.4 g of o-methyl-benzoyl chloride and 21 g of dichloroethane to the materials, while maintain a constant temperature of 10°C or less, add dropwise the above-mentioned solution of o-methyl-benzoyl chloride and dichloroethane over to the materials for about 1.5 hours, continue to agitate the materials in the flask for 2 hours; then add 25.4 g of AlCl₃ (fine grinding) to the materials; while maintaining a constant temperature of 10°C or less, add dropwise the solution of 42.2 g of 3-cyclopentyl propionyl chloride and 20 g of dichloroethane to the materials over for about 1.5 hours; the temperature rises to 15°C, continue to agitate the materials for 2 hours. Finally, discharge the materials.

### Post-treatment:

Under agitation, gradually add the above-mentioned materials into the diluted solution of hydrochloric acid, which is prepared by mixing 65 ml of concentrated hydrochloric acid with 400 g of ice; separate out the lower layer of the mixture with a separating funnel; extract the upper layer of the mixture with 50 ml of dichloroethane; mix the lower layer materials with the extracted materials to obtain a mixture; wash it with the solution of sodium bicarbonate, which is prepared by mixing 10 g of sodium bicarbonate with 200 g of water, furthermore, wash it with 200 ml of water three times, till the mixture have pH 7; subsequently, remove water with 30 g of anhydrous magnesium sulfate, then remove dichloroethane via rotary evaporation method; after the evaporation, the crude product in distilling flask is a powdery solid, pour the powdery solid into 200 ml of light petroleum which is distilled at normal pressure to obtain a mixture; filtrate the mixture with air pump filtration to obtain a filter cake; pour the filter cake into 150 ml of anhydrous ethanol to obtain a mixture; heat the mixture to reflux, then, cool it down to room temperature, furthermore, freeze it for 2 hours; finally, filtrate it with air pump filtration to obtain a powdery white solid, dry the powdery solid in an oven at 75 °C for 2 hours to obtain the product, The weight is 50.3 g; the purity is more than 96 %.

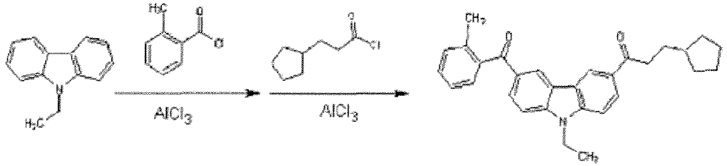

### Step 2: Preparation of 1-(6-o-methylbenzoyl-9-ethylcarbazole-3-yl)-(3-cyclopentyl)-propane-1,2-dione-2-oxime

Charge 45 g of the product obtained in the step 1, 450 g of tetrahydrofuran, 225 g of concentrated hydrochloric acid, and 19.2 g of isoamyl into a 500 ml four-neck flask, agitate them at room temperature for 5 hours. Finally, discharge the materials. During the reaction process, large quantities of the materials were precipitated.

### Post-treatment:

Pour the above-mentioned materials into a big beaker, furthermore, pour 1000g of water into the same beaker, carry on agitating to obtain a mixture; leave the mixture at rest overnight; then the mixture is separated to two layers, separate the layers to obtain viscous yellow liquid, extract it with dichloroethane; in order to remove water from the materials, pour 50 g of anhydrous magnesium sulfate into them; carry on filtrating to obtain a filtrate; evaporate the filtrate via rotary evaporation method; after the evaporation, oily viscous material is obtained in distilling flask. The weight is 50 g; and the purity is more than 90 %.

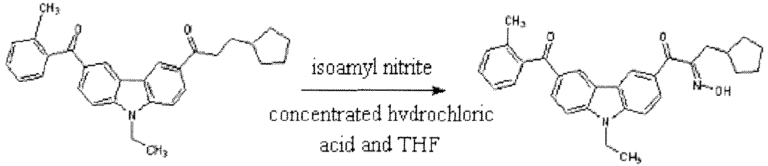

### Step 3: Preparation of 1-(6-o-methylbenzoyl-9-ethylcarbazole-3-yl)-(3-cyclopentyl)-propane-1,2-dione-2-beozoic acid oxime ester

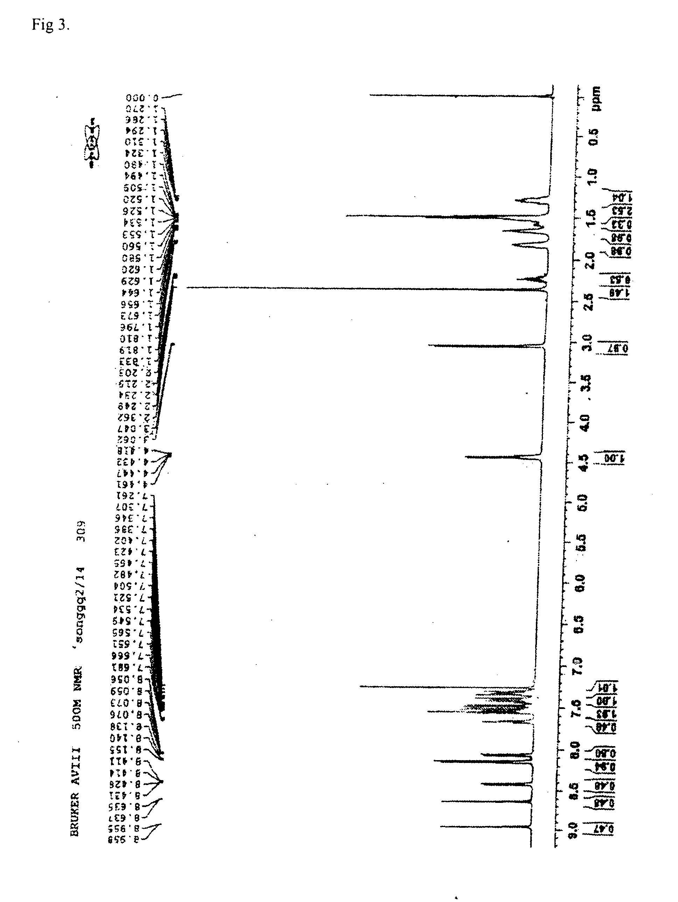

Charge 50 g of the product obtained in the step 2, 350 ml of dichloroethane and 16.2 g of triethylamine into a 1000 ml four-neck flask, carry on agitating, cool down the materials in the flask with an ice bath; when the temperature falls to 0°C, begin to add dropwise the solution of 16.5 g of benzoyl chloride and 15 g of dichloroethane to the materials for about 1.5 hours; continue to agitate the materials for 1 hour; then add dropwise 500 ml of cold water to the materials; separate out organic liquid layer of the materials with a separating funnel, wash it with 200 ml of 5% sodium bicarbonate one time, furthermore, it with 200 ml of water two times till the organic liquid layer have pH 7; after that, wash it with the diluted solution of hydrochloric acid one time, which is prepared by mixing 20g of concentrated hydrochloric acid with 400 ml of water; then wash it with 200 ml of water three times; subsequently, in order to remove water, pour 100 g of anhydrous magnesium sulfate into it; distill out the solvents via rotary evaporation method to obtain a viscous liquid; add proper quantity of methanol into the viscous liquid, then a white solid product as a precipitate is obtained; finally, carry on filtrating and drying to obtain a final product. The weight is 35.2 g; the yield is equal to 60 %; and the purity is more than 98 %. According to the ¹HNMR spectrogram as shown in Fig. 3, it can be deduced that the structural formula of the final product of this example is as follows:

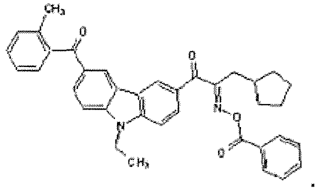

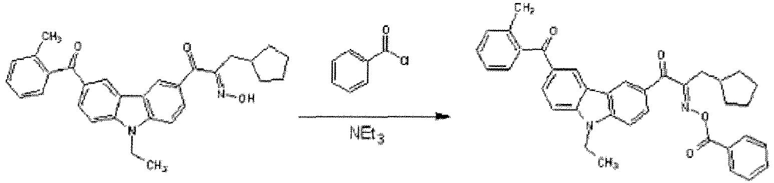

Comparing UV absorption spectrum of 1-(4-phenylthiophenyl)-(3-cyclopentyl)-propane-1,2-dione-2-benzoic acid oxime ester B which is prepared in example 1, 1-(6-o-methylbenzoyl-9-ethylcarbazole-3-yl)-(3-cyclopentyl)-propane-1,2-dione-2-benzoic acid oxime ester C which is prepared in example 2, and 1-(4-phenylthiophenyl)-n-octane-1,2-dione-2-benzoic acid oxime ester (i.e. OXE-1), as shown in Fig.1, the ketoxime ester compounds have same or similar UV absorption spectrum in the case of having same weight concentration. Wherein, the UV absorption spectrum of materials A and B are lapped. But material B obviously has a better thermal stability than that of material A. The UV absorption spectrum of material C differ from that of A, it shows obvious red shift and has biggish absorption peak at 365nm. Material C obviously has a better thermal stability than that of material A. As shown in the follow tables, the practical performance (sensitivity, thermal stability, solubility) of the new ketoxime ester of the present invention is superior to that of OXE-1 and OXE-2.

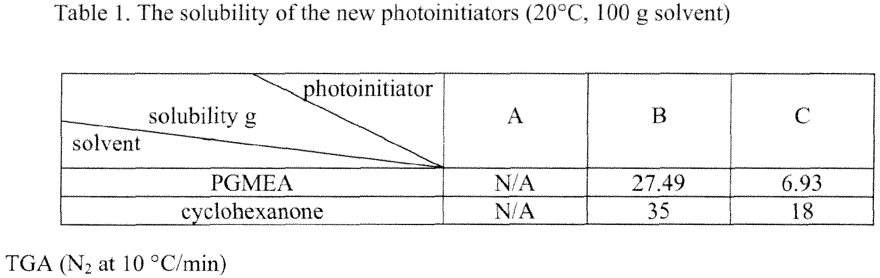

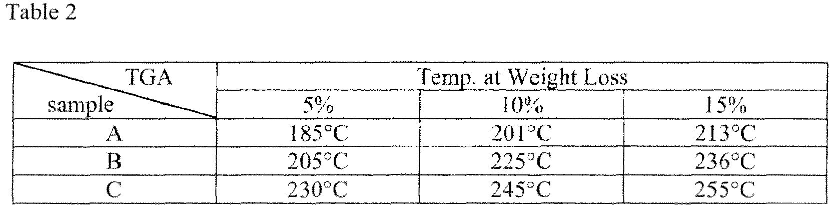

## Claims

1. A compound shown in the following general formula: 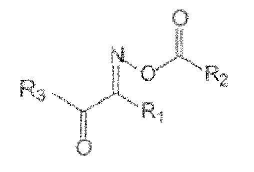 wherein, the group R₁ is 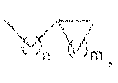 wherein, n is the integer of 1∼4, m is the integer of 1∼6; R₂ is C1-8 alkyl, phenyl, substituted phenyl, benzyl, or substituted benzyl; R₃ is diphenylthioether group, substituted diphenylthioether group, carbazyl or substituted carbazyl.

2. The compound according to claim 1, **characterized in that** R₁ is cyclopentylmethylene, R₃ is 6-o-methyl-benzoyl-9-ethylcarbazyl group, R₂ is phenyl, the structural formula is

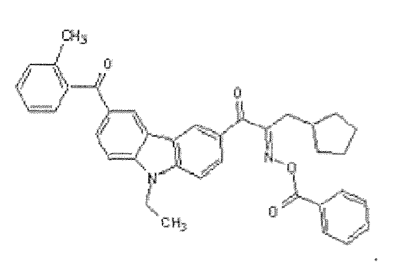

3. A process for preparing the ketoxime ester photoinitiator according to claim 1, **characterized in that** it comprises the following steps:
a. preparation of intermediate I: diphenylthioether, substituted diphenylthioether, carbazole or substituted carbazole as a starting material is mixed with an acyl halide compound having R₁ group, with the participant of aluminum chloride or zinc chloride, an Friedel-Crafts Reaction is carried out, by which a ketoxime ester photoinitiator intermediate I is obtained.
b. preparation of intermediate II: the intermediate I is oxidized by isoamyl nitrite, with the catalysis of hydrogen chloride, sodium alcoholate or potassium alcoholate, a hydroxylamine compound, i.e. intermediate II, is obtained.
c. preparation of the compound the intermediate II is esterified by acyl halide compound having R₂ group or anhydride having R₂ group to prepare ketoxime ester photoinitiator product.

4. The process for preparing the compound according to claim 3, **characterized in that** the structure of the acyl halide compound having R₁ group is 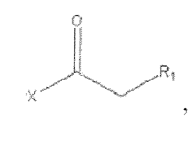 the structure of the intermediate I is 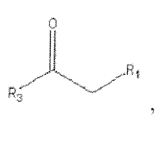 the structure of the intermediate II is 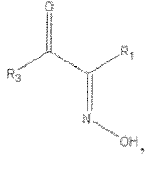 the structure of the acy) halide compound having R₂ group is 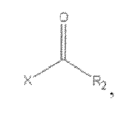 wherein, the structure of R₁ is 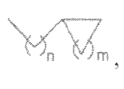 wherein, n is an integer of anyone of 1∼4, m is an of anyone of 1∼6; R₃ is diphenylthioether group, substituted diphenylthioether group, carbazyl or substituted carbazyl.

5. The process for preparing the compound according to claim 3, **characterized in that** the specific process of preparing intermediate I of the step a is as follows: the starting material, AlCl₃, and organic solvent A are mixed by agitating, pump the argon gas into the mixture to protect the reaction system, cool down them with an ice bath; when the temperature falls to 0°C, add dropwise a mixture solution of acyl halide compound having R₁ group and organic solvent A for 1.5∼2 hours, while maintain a constant temperature or 0∼10 °C; then the temperature rises to 5∼35 °C, continue to agitate the reaction mixture for 1.5∼3 hours; finally, discharge the materials, after post-treatment the intermediate I as a sheet white solid is obtained; therein, the optimal mole ratio of the starting material, AlCl₃ and the acyl halide compound having R₁ group is 1:1:1.

6. The process for preparing the compound according to claim 5, **characterized in that** said organic solvent A is dichloroethane, dichloromethane or carbon tetrachloride.

7. The process for preparing the compound according to claim 3, **characterized in that** the process of preparing intermediate II of the step b is as follows: add hydrogen chloride, sodium alcoholate or potassium alcoholate into a mixture solution of the intermediate I obtained from the step a, the organic solvent B and isoamyl nitrite, continue to agitate the reaction mixture for 5∼6 hours at room temperature; after post-treatment the intermediate II as a powdery white solid is obtained; wherein, the optimal mole ratio of the intermediate I to isoamyl nitrite is 1:1.6.

8. The process for preparing the compound according to claim 7, **characterized in that** said organic solvent B is dichloroethane or tetrahydrofuran.

9. The process for preparing the compound according to claim 3, **characterized in that** the process of preparing the ketoxime ester photoinitiator of the step c is as follows: the intermediate II, dichloroethane and triethylamine are mixed by agitating, cool down them with an ice bath; when the temperature to 0°C, begin to add dropwise a mixture solution of acyl halide compound having R₂ group and dichloroethane for 1∼1.5 hours, continue to agitate the reaction mixture for 1∼2 hours; then add cold water dropwise to the materials; separate out organic liquid layer of the materials with a separating funnel; wash it with 5% sodium bicarbonate one time, then wash it with water till the organic liquid layer have pH 7; furthermore, wash it with diluted hydrochloric acid solution one time, and then wash it with water three times; subsequently, in order to remove water, pour anhydrous magnesium sulfate into it; distill out the solvents via rotary evaporation method to obtain a viscous liquid; pour methanol into it to obtain a white solid product as a precipitate.

10. Use of the compound according to claim 2 as photoinitiator in LED cold light source.

## Patentansprüche

1. Verbindung, die in der folgenden allgemeinen Formel gezeigt ist: 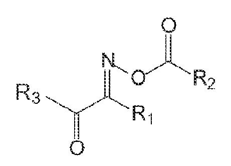 wobei die Gruppe R₁ 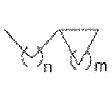 ist, wobei n eine ganze Zahl von 1-4 ist, m eine ganze Zahl von 1-6 ist, R₂ ein C1-8-Alkyl, Phenyl, substituiertes Phenyl, Benzyl oder substituiertes Benzyl ist, R₃ eine Diphenylthioethergruppe, eine substituierte Diphenylthioethergruppe, Carbazyl oder substituiertes Carbazyl ist.

2. Verbindung nach Anspruch 1, **dadurch gekennzeichnet, dass** R₁ Cyclopentylmethylen, R₃ eine 6-o-Methyl-Benzoyl-9-Ethylcarbazylgruppe, R₂ Phenyl, und die Strukturformel 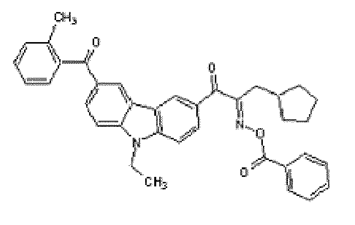 ist.

3. Verfahren zum Zubereiten der Verbindung nach Anspruch 1, **dadurch gekennzeichnet, dass** es die folgenden Schritte umfasst:
a. Zubereiten des Zwischenproduktes I: Diphenylthioether, substituierter Diphenylthioether, Carbazol oder substituiertes Carbazol wird als Startmaterial mit einer Acylhalidverbindung, die eine R₁-Gruppe aufweist, unter Beteiligung von Aluminiumchlorid oder Zinkchlorid gemischt, eine Friedel-Crafts-Reaktion wird ausgeführt, durch die ein Ketoximesterfotoinitiator-Zwischenprodukt I erhalten wird,
b. Zubereiten des Zwischenproduktes II: Das Zwischenprodukt I wird unter Katalyse von Chlorwasserstoff, Natriumalkoholat oder Kaliumalkoholat durch Isoamylnitrit oxidiert, eine Hydroxylaminverbindung, d.h. Zwischenprodukt II, wird erhalten,
c. Zubereitung der Verbindung: Das Zwischenprodukt II wird durch eine Acylhalidverbindung, die eine R₂-Gruppe aufweist, oder ein Anhydrid, das eine R₂-Gruppe aufweist, verestert, um das Ketoximesterfotoinitiatorprodukt zuzubereiten.

4. Verfahren zum Zubereiten der Verbindung nach Anspruch 3, **dadurch gekennzeichnet, dass** die Struktur der Acylhalidverbindung, die eine R₁-Gruppe aufweist, 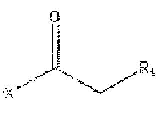 ist, die Struktur des Zwischenproduktes I 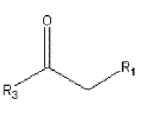 ist, die Struktur des Zwischenproduktes II 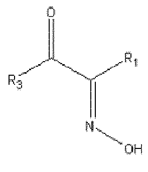 ist, die Struktur der Acylhalidverbindung, die eine R₂-Gruppe aufweist, 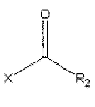 ist, wobei die Struktur von R₁ 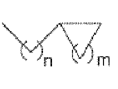 ist, wobei n eine ganze Zahl von 1-4 ist, m eine ganze Zahl von 1-6 ist, R₃ eine Diphenylthioethergruppe, eine substituierte Diphenylthioethergruppe, ein Carbazyl oder ein substituiertes Carbazyl ist.

5. Verfahren zum Zubereiten der Verbindung nach Anspruch 3, **dadurch gekennzeichnet, dass** das spezifische Verfahren zum Zubereiten des Zwischenproduktes I in Schritt a. wie folgt ist: Das Startmaterial, AlCl₃ und ein organisches Lösungsmittel A werden durch Rühren gemischt, Argongas wird in die Mischung gepumpt, um das Reaktionssystem zu schützen, es wird mit einem Eisbad heruntergekühlt; wenn die Temperatur auf 0° C fällt, wird tröpfchenweise eine Mischlösung aus einer Acylhalidverbindung, die eine R₁-Gruppe aufweist, und einem organischen Lösungsmittel A für 1,5 bis 2 Stunden zugefügt, während eine konstante Temperatur von 0 bis 10° C gehalten wird; dann wird die Temperatur auf 5 bis 35° C angehoben, Rühren der Reaktionsmischung wird für 1,5 bis 3 Stunden fortgesetzt; schlussendlich werden die Materialien nach einer Nachbehandlung des Zwischenproduktes I freigesetzt, wenn ein weißer plättchenartiger Feststoff erhalten wird, wobei das optimale Molverhältnis von Startmaterial, AlCl₃ und der Acylhalidverbindung, die eine R₁-Gruppe aufweist, 1 : 1 : ist.

6. Verfahren zum Zubereiten der Verbindung nach Anspruch 5, **dadurch gekennzeichnet, dass** das organische Lösungsmittel A Dichlorethan, Dichlormethan oder Kohlenstofftetrachlorid ist.

7. Verfahren zum Zubereiten der Verbindung nach Anspruch 3, **dadurch gekennzeichnet, dass** das Verfahren zum Zubereiten des Zwischenproduktes II in Schritt b. wie folgt ist: Hinzufügen von Chlorwasserstoff, Natriumalkoholat oder Kaliumalkoholat zu einer Mischlösung aus dem in Schritt a. erhaltenen Zwischenprodukt I, Fortsetzen des Rührens der Reaktionsmischung für 5 bis 6 Stunden bei Raumtemperatur, Nachbehandlung des Zwischenproduktes II, wenn ein pulvriger weißer Feststoff erhalten wird, wobei das optimale Molverhältnis von Zwischenprodukt I zu Isoamylnitrit 1 : 1,6 ist.

8. Verfahren zum Zubereiten der Verbindung nach Anspruch 7, **dadurch gekennzeichnet, dass** das organische Lösemittel B Dichlorethan oder Tetrahydrofuran ist.

9. Verfahren zum Zubereiten der Verbindung nach Anspruch 3, **dadurch gekennzeichnet, dass** das Verfahren zum Zubereiten des Ketoximesterfotoinitiatorproduktes in Schritt c. wie folgt ist: Das Zwischenprodukt II, Dichlorethan und Triethylamin werden durch Rühren gemischt, in einem Eisbad abgekühlt; wenn die Temperatur auf 0°C fällt, wird die tröpfchenweise Zugabe einer Mischlösung aus einer Acylhalidverbindung, die eine R₂-Gruppe aufweist, und Dichlorethan für 1 bis 1,5 Stunden begonnen, Fortsetzen des Rührens der Reaktionsmischung für 1 bis 2 Stunden, dann tröpfchenweises Hinzufügen von kaltem Wasser zu den Materialien, Abtrennen der organischen Flüssigkeitsschicht der Materialien mit einem Trenntrichter, einmaliges Waschen mit 5 % Natriumbikarbonat, dann Waschen mit Wasser bis die organische Flüssigkeitsschicht einen pH-Wert von 7 aufweist; ferner einmaliges Waschen mit verdünnter Salzsäurelösung und dann dreimaliges Waschen mit Wasser, anschließend, um Wasser zu entfernen, Eingießen von Magnesiumsulfat, Ausdestillieren des Lösungsmittels mittels eines Rotationsverdunstungsverfahrens, um eine viskose Flüssigkeit zu erhalten, Eingießen von Methanol, um ein weißes Feststoffprodukt als ein Präzipitat zu erhalten.

10. Verwendung der Verbindung nach Anspruch 2 als Fotoinitiator in einer LED-Kaltlichtquelle.

## Revendications

1. Composé montré dans la formule générale suivante : 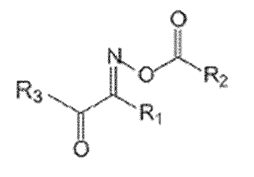 où le groupe R₁ est 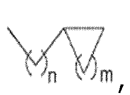 où n est l'entier de 1-4, m est l'entier de 1-6 ; R₂ est C1-8 alkyle, phényle, phényle substitué, benzyle ou benzyle substitué ; R₃ est un groupe diphénylthioéther, un groupe diphénylthioéther substitué, carbazyle ou carbazyle substitué.

2. Composé selon la revendication 1, **caractérisé en ce que** R₁ est cyclopentylméthylène, R₃ est un groupe 6-o-méthyl-benzoyl-9-éthylcarbazyle, R₂ est phényle, la formule structurale est

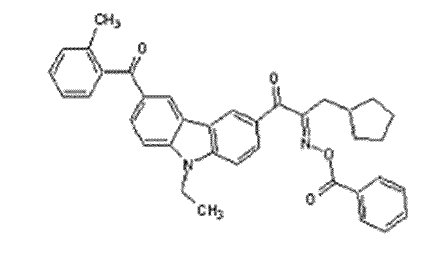

3. Procédé pour préparer le composé selon la revendication 1, **caractérisé en ce qu'**il comprend les étapes suivantes :
a. préparation de l'intermédiaire I : un diphénylthioéther, un diphénylthioéther substitué, un carbazole ou un carbazole substitué comme produit de départ est mélangé avec un composé halogénure d'acyle ayant un groupe R₁, avec la participation de chlorure d'aluminium ou de chlorure de zinc, une réaction de Friedel-Crafts est réalisée, par laquelle un intermédiaire I de photoinitiateur ester de cétoxime est obtenu,
b. préparation de l'intermédiaire II : l'intermédiaire I est oxydé par le nitrite d'isoamyle, avec la catalyse de chlorure d'hydrogène, d'un alcoolate de sodium ou d'un alcoolate de potassium, un composé d'hydroxylamine, c'est-à-dire l'intermédiaire II, est obtenu,
c. préparation du composé : l'intermédiaire II est estérifié par un composé halogénure d'acyle ayant un groupe R₂ ou un anhydride ayant un groupe R₂ pour préparer un produit photo-initiateur ester de cétoxime.

4. Procédé pour préparer le composé selon la revendication 3, **caractérisé en ce que** la structure du composé halogénure d'acyle ayant un groupe R₁ est 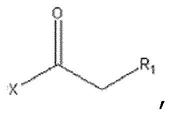 la structure de l'intermédiaire I est 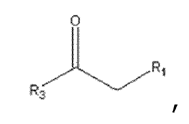 la structure de l'intermédiaire II est 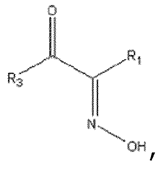 la structure du composé halogénure d'acyle ayant un groupe R₂ est 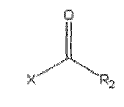 où la structure de R₁ est 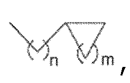 où n est un entier de l'un quelconque de 1-4, m est un entier de l'un quelconque de 1-6 ; R₃ est un groupe diphénylthioéther, un groupe diphénylthioéther substitué, carbazyle ou carbazyle substitué.

5. Procédé pour préparer le composé selon la revendication 3, **caractérisé en ce que** le procédé spécifique de préparation de l'intermédiaire I de l'étape a est le suivant : le produit de départ, AlCl₃ et un solvant organique A sont mélangés par agitation, pomper le gaz argon dans le mélange pour protéger le système réactionnel, les refroidir avec un bain de glace ; quand la température baisse jusqu'à 0°C, ajouter goutte à goutte une solution mélangée de composé halogénure d'acyle ayant un groupe R₁ et de solvant organique A pendant 1,5-2 h, tout en maintenant une température constante de 0-10°C ; puis la température monte à 5-35°C, continuer à agiter le mélange réactionnel pendant 1,5-3 h, finalement, évacuer les produits, après un post-traitement l'intermédiaire I sous forme d'un solide blanc en feuille est obtenu ; où le rapport molaire optimal du produit de départ, de AlCl₃ et du composé halogénure d'acyle ayant un groupe R₁ est 1:1:1.

6. Procédé pour préparer le composé selon la revendication 5, **caractérisé en ce que** ledit solvant organique A est le dichloroéthane, le dichlorométhane ou le tétrachlorure de carbone.

7. Procédé pour préparer le composé selon la revendication 3, **caractérisé en ce que** le procédé de préparation de l'intermédiaire II de l'étape b est le suivant : ajouter du chlorure d'hydrogène, un alcoolate de sodium ou un alcoolate de potassium dans une solution mélangée de l'intermédiaire I obtenu à partir de l'étape a, du solvant organique B et du nitrite d'isoamyle, continuer à agiter le mélange réactionnel pendant 5-6 h à la température ambiante ; après post-traitement l'intermédiaire II sous forme d'un solide blanc pulvérulent est obtenu ; où le rapport molaire optimal de l'intermédiaire I au nitrite d'isoamyle est 1:1,6.

8. Procédé pour préparer le composé selon la revendication 7, **caractérisé en ce que** ledit solvant organique B est le dichloroéthane ou le tétrahydrofurane.

9. Procédé pour préparer le composé selon la revendication 3, **caractérisé en ce que** le procédé de préparation du photo-initiateur ester de cétoxime de l'étape c est le suivant : l'intermédiaire II, le dichloro-éthane et la triéthylamine sont mélangés par agitation, les refroidir avec un bain de glace, quand la température baisse jusqu'à 0°C, commencer à ajouter goutte à goutte une solution mélangée de composé halogénure d'acyle ayant un groupe R₂ et de dichloroéthane pendant 1-1,5 h, continuer à agiter le mélange réactionnel pendant 1-2 h, puis ajouter de l'eau froide goutte à goutte aux produits ; séparer la couche liquide organique des produits avec un entonnoir à décantation ; la laver avec du bicarbonate de sodium à 5 % une fois, puis la laver avec de l'eau jusqu'à ce que la couche liquide organique ait un pH de 7 ; en outre, la laver avec une solution diluée d'acide chlorhydrique une fois, puis la laver avec de l'eau trois fois ; ensuite, pour retirer l'eau, verser dans celle-ci du sulfate de magnésium anhydre ; chasser les solvants par distillation via le procédé d'évaporation rotative pour obtenir un liquide visqueux ; verser du méthanol dans celui-ci pour obtenir un produit solide blanc sous forme d'un précipité.

10. Utilisation du composé selon la revendication 2 comme photo-initiateur dans une source de lumière froide DEL.
